# EUROPEAN PATENT SPECIFICATION

(11) **EP 1 567 090 B1**
(45) Date of publication and mention of the grant of the patent: **15.05.2013**
(21) Application number: 03783488.4
(22) Date of filing: 12.11.2003
(51) Int. Cl.: A61F 2/06

(54) **PHOTO CURABLE ENDOPROSTHESIS**
LICHTHÄRTBARE ENDOPROTHESE
ENDOPROTHESES PHOTODURCISSABLES

(30) Priority: 15.11.2002 US 426734 P; 15.01.2003 US 342771
(43) Date of publication of application: 31.08.2005
(73) Proprietor: Synecor, LLC, Durham, NC 27703 (US)
(72) Inventor: WILLIAMS, Michael, S., Santa Rosa, CA 95404 (US); HOLBROOK, Kevin, D., Cloverdale, CA 95425 (US); GLENN, Richard, A., Santa Rosa, CA 95409 (US); SMITH, Jeffrey, A., Santa Rosa, CA 95404 (US); DESIMONE, Joseph, M., Chapel Hill, NC 27516 (US)
(74) Representative: Chalk, Anthony John
(86) International application number: PCT/US2003/036422
(87) International publication number: WO 2004/045462

(56) References cited:
- EP-A1- 0 732 084
- WO-A1-95/07667
- WO-A1-98/40034
- US-A- 5 100 429
- US-A- 5 464 419
- US-A- 5 591 199
- US-A- 5 591 199
- US-A1- 2002 111 673
- US-A1- 2003 044 514

## Description

### FIELD OF THE INVENTION

The invention herein relates generally to medical devices and the manufacture thereof, and more particularly to improved endoprostheses for use in the treatment of strictures in lumens of the body.

### BACKGROUND OF THE INVENTION

Ischemic heart disease is the major cause of death in industrialized countries. Ischemic heart disease, which often results in myocardial infarction, is a consequence of coronary atherosclerosis. Atherosclerosis is a complex chronic inflammatory disease and involves focal accumulation of lipids and inflammatory cells, smooth muscle cell proliferation and migration, and the synthesis of extracellular matrix. Nature, 1993; 362: 801-809. These complex cellular processes result in the formation of atheromatous plaque, which consists of a lipid-rich core covered with a collagen-rich fibrous cap, varying widely in thickness. Further, plaque disruption is associated with varying degrees of internal hemorrhage and luminal thrombosis because the lipid core and exposed collagen are thrombogenic. J. Am. Coll. Cardiol., 1994; 23: 1562-1569. Acute coronary syndrome usually occurs as a consequence of such disruption or ulceration of a so called "vulnerable plaque". Arterioscler. Thromb. Vasc. Biol., Volume 22, No. 6, June 2002, p. 1002.

In addition to coronary bypass surgery, a current treatment strategy to alleviate vascular occlusion includes percutaneous transluminal coronary angioplasty, expanding the internal lumen of the coronary artery with a balloon. Roughly 800,000 angioplasty procedures are performed in the U.S. each year (Arteriosclerosis, Thrombosis, and Vascular Biology, Volume 22, No. 6, June 2002, p. 884). However, 30% to 50% of angioplasty patients soon develop significant restenosis, a narrowing of the artery through migration and growth of smooth muscle cells.

In response to the significant restenosis rate following angioplasty, percutaneously placed endoprostheses have been extensively developed to maintain fluid flow through a diseased coronary artery. Such endoprostheses, or stents, which have been traditionally fabricated using metal alloys, include self-expanding or balloon-expanded devices that are "tracked" through the vasculature and deployed proximate one or more lesions. Stents considerably enhance the long-term benefits of angioplasty, but 10% to 50% of patients receiving stents still develop restenosis (J. Am. Coll. Cardiol., 2002; 39: 183-193. Consequently, a significant portion of the relevant patient population undergoes continued monitoring and, in many cases, additional treatment.

EP-A-732084 relates to a catheter comprising a tube-like basic body with a proximal and a distal end. The catheter is provided with a single lumen and a balloon member made of light-transmitting material. The balloon member is arranged with an end-section to the distal end of the basic body and the opposite, relatively distal end-section thereof is closed. An assembly of a catheter and a flexible rod-shaped light conductor which comprises light conducting material and has a smaller diameter than the lumen of the basic body and a greater length than the catheter is also disclosed.

WO-A-95/07667 teaches a balloon for enlarging *in situ* to fit against a vessel wall and harden in place on application of energy. The balloon may have portions of scission material which will degrade upon application of energy, thereby allowing the end portions to detach from the balloon and to adhere to a shaft while the body of the balloon remains in the vessel.

US-A-5591199 discloses a stent for supporting a selected portion of a body lumen, comprising a fibrous material which is treated with a curable material to form a curable fiber composite. The fiber composite is positioned within a body lumen and, upon curing of the curable material, forms a rigid support structure.

US-A-5100429 is concerned with a system for the delivery of an uncured or partially cured, collagen-based material to a selected site in a blood vessel and its crosslinking in the blood vessel by laser energy or other suitable energy to form an endovascular stent. The collagen-based material can be delivered to the blood vessel as a coating on an inflatable balloon mounted on the distal end of a catheter, or in liquid form forced through a porous balloon to form a tubular configuration.

WO-A-98140034 describes a stent which is created *in situ* at a site in a blood vessel from a flowable material which is delivered in a porous, flexible angioplasty balloon. The flowable material is extruded in place into a mold cavity formed by the porous surface of the angioplasty balloon and the surrounding endovascular wall and energy is applied to solidify the material and to interlink the stent material with fissures and breaks in the endovascular wall. The balloon is then withdrawn leaving a fully formed stent having a central lumen for enhanced blood flow.

US-A-2002/111673 discloses tailored, biocompatible conduits for use as stents, vascular grafts, and drug delivery devices, and methods of making the conduits. The conduits are formed from a resin-impregnated matrix that is introduced to a site in a malleable state, formed into a desired shape, and cured by exposure to radiant energy.

Continued improvements in stent technology aim at producing easily tracked, easily visualized and readily deployed stents, which exhibit the requisite radial strength without sacrificing a small delivery profile and sufficient flexibility to traverse the diseased human vasculature. Further, numerous therapies directed to the cellular mechanisms of accumulation of inflammatory cells, smooth muscle cell proliferation and migration show tremendous promise for the successful long-term treatment of ischemic heart disease. Consequently, advances in coupling delivery of such therapies to the mechanical support of vascular endoprostheses, delivered proximate the site of disease, offer great hope to the numerous individuals suffering heart disease.

While advances in the understanding of ischemic heart disease as a complex chronic inflammatory process take place, traditional diagnostic techniques such as coronary angiography yield to next generation imaging modalities. In fact, coronary angiography may not be at all useful in identifying inflamed atherosclerotic plaques that are prone to producing clinical events. Imaging based upon temperature differences, for example, are undergoing examination for use in detecting coronary disease. Magnetic resonance imaging (MRI) is currently emerging as the state of the art diagnostic arterial imaging, enhancing the detection, diagnosis and monitoring of the formation of vulnerable plaques. Transluminal intervention guided by MRI is expected to follow. However, metals produce distortion and artifacts in MR images, rendering use of the traditionally metallic stents in coronary, biliary, esophageal, ureteral, and other body lumens incompatible with the use of MRI.

Consequently, an emerging clinical need for interventional devices that are compatible with and complementary to new imaging modalities is evident. Further, devices that exhibit improved trackability to previously undetectable disease within remote regions of the body, especially the coronary vasculature are needed. And finally, devices that both exhibit improved mechanical support and are readily compatible with adjunct therapies in order to lower or eliminate the incidence of restenosis are needed.

### SUMMARY OF THE INVENTION

The invention provides a system for treating a stenosis in a body lumen, said system comprising:
an endoprosthesis (10, 30, 40) comprising a first curable material (15);
means for selectively curing said first curable material,
wherein said first curable material is curable by exposure to a radiation source, said system further comprising a radiation source, wherein said radiation source comprises a radiation emitting catheter (18),
characterised in that said endoprosthesis further comprises a first region, said means for selectively curing said first curable material comprises a radiation emitting catheter comprising an inflatable balloon (35, 45), wherein said balloon comprises means to prevent the exposure of said first region of said endoprosthesis to radiation and wherein said means to prevent the exposure of said first region to radiation comprises a photolithographic coating (32).

### BRIEF DESCRIPTION OF THE DRAWINGS

**FIGS. 1A-B** are plan views of an embodiment according to the invention during and after photocuring.
**FIG. 2** is a plan view of alternative embodiment according to the invention.
**FIG. 3** is a plan view of yet another embodiment according to the invention.
**FIG. 4** is a plan view of still another embodiment according to the invention.
FIGS. 5A-C illustrate a series of steps in the manufacture of an alternative embodiment according to the invention.

### DETAILED DESCRIPTION OF THE INVENTION

Although the invention herein is not limited as such, some embodiments of the invention comprise materials that are erodible. "Erodible" refers to the ability of a material to maintain its structural integrity for a desired period of time, and thereafter gradually undergo any of numerous processes whereby the material substantially loses tensile strength and mass. Examples of such processes comprise hydrolysis, enzymatic and non-enzymatic degradation, oxidation, enzymatically-assisted oxidation, and others, thus including bioresorption, dissolution, and mechanical degradation upon interaction with a physiological environment into components that the patient's tissue can absorb, metabolize, respire, and/or excrete. Polymer chains are cleaved by hydrolysis and are eliminated from the body through the Krebs cycle, primarily as carbon dioxide and in urine. "Erodible" and "degradable" are intended to be used interchangeably herein.

The term "endoprosthesis" refers to any prosthetic device placed within a body lumen or duct to in order to therapeutically treat the body lumen or duct, including but limited to the objective of restoring or enhancing flow of fluids through a body lumen or duct.

A "self-expanding" endoprosthesis has the ability to revert readily from a reduced profile configuration to a larger profile configuration in the absence of a restraint upon the device that maintains the device in the reduced profile configuration.

"Balloon expandable" refers to a device that comprises a reduced profile configuration and an expanded profile configuration, and undergoes a transition from the reduced configuration to the expanded configuration via the outward radial force of a balloon expanded by any suitable inflation medium

The term "balloon assisted" refers to a self-expanding device the final deployment of which is facilitated by an expanded balloon.

The term "fiber" refers to any generally elongate member fabricated from any suitable material, whether polymeric, metal or metal alloy, natural or synthetic.

As used herein, a device is "implanted" if it is placed within the body to remain for any length of time following the conclusion of the procedure to place the device within the body.

The term "diffusion coefficient" refers to the rate by which a substance elutes, or is released either passively or actively from a substrate.

As used herein, the term "braid" refers to any braid or mesh or similar woven structure produced from between 1 and several hundred longitudinal and/or transverse elongate elements woven, braided, knitted, helically wound, or intertwined any manner, at angles between 0 and 180 degrees and usually between 45 and 105 degrees, depending upon the overall geometry and dimensions desired.

Unless specified, suitable means of attachment may include by melt bond, chemical bond, adhesive, sintering, welding, or any means known in the art.

"Shape memory" refers to the ability of a material to undergo structural phase transformation such that the material may define a first configuration under particular physical and/or chemical conditions, and to revert to an alternate configuration upon a change in those conditions. Shape memory materials may be metal alloys including but not limited to nickel titanium, or may be polymeric. A polymer is a shape memory polymer if the original shape of the polymer is recovered by heating it above a shape recovering temperature (defined as the transition temperature of a soft segment) even if the original molded shape of the polymer is destroyed mechanically at a lower temperature than the shape recovering temperature, or if the memorized shape is recoverable by application of another stimulus. Such other stimulus may include but is not limited to pH, salinity, hydration, and others. Some embodiments according to the invention may comprise one or more polymers having a structure that assumes a first configuration, a second configuration, and a hydrophilic polymer of sufficient rigidity coated upon at least a portion of the structure when the device is in the second configuration. Upon placement of the device in an aqueous environment and consequent hydration of the hydrophilic polymer, the polymer structure reverts to the first configuration.

As used herein, the term "segment" refers to a block or sequence of polymer forming part of the shape memory polymer. The terms hard segment and soft segment are relative terms, relating to the transition temperature of the segments. Generally speaking, hard segments have a higher glass transition temperature than soft segments, but there are exceptions. Natural polymer segments or polymers include but are not limited to proteins such as casein, gelatin, gluten, zein, modified zein, serum albumin, and collagen, and polysaccharides such as alginate, chitin, celluloses, dextrans, pullulane, and polyhyaluronic acid; poly(3-hydroxyalkanoate)s, especially poly(.beta.-hydroxybutyrate), poly(3-hydroxyoctanoate) and poly(3-hydroxyfatty acids).

Representative natural erodible polymer segments or polymers include polysaccharides such as alginate, dextran, cellulose, collagen, and chemical derivatives thereof (substitutions, additions of chemical groups, for example, alkyl, alkylene, hydroxylations, oxidations, and other modifications routinely made by those skilled in the art), and proteins such as albumin, zein and copolymers and blends thereof, alone or in combination with synthetic polymers.

Suitable synthetic polymer blocks include polyphosphazenes, poly(vinyl alcohols), polyamides, polyester amides, poly(amino acid)s, synthetic poly(amino acids), polyanhydrides, polycarbonates, polyacrylates, polyalkylenes, polyacrylamides, polyalkylene glycols, polyalkylene oxides, polyalkylene terephthalates, polyortho esters, polyvinyl ethers, polyvinyl esters, polyvinyl halides, polyvinylpyrrolidone, polyesters, polylactides, polyglycolides, polysiloxanes, polyurethanes and copolymers thereof.

Examples of suitable polyacrylates include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate) and poly(octadecyl acrylate).

Synthetically modified natural polymers include cellulose derivatives such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, arboxymethyl cellulose, cellulose triacetate and cellulose sulfate sodium salt. These are collectively referred to herein as "celluloses".

Examples of synthetic degradable polymer segments or polymers include polyhydroxy acids, such as polylactides, polyglycolides and copolymers thereof; poly(ethylene terephthalate); poly(hydroxybutyric acid); poly(hydroxyvaleric acid); poly[lactide-co-(.epsilon.-caprolactone)]; poly[glycolide-co-(.epsilon-caprolactone)]; polycarbonates, poly(pseudo amino acids); poly(amino acids); poly(hydroxyalkanoate)s; polyanhydrides; polyortho esters; and blends and copolymers thereof.

For those embodiments comprising a shape memory polymer, the degree of crystallinity of the polymer or polymeric block(s) is between 3 and 80%, more often between 3 and 65%. The tensile modulus of the polymers below the transition temperature is typically between 50 MPa and 2 GPa (gigapascals), whereas the tensile modulus of the polymers above the transition temperature is typically between 1 and 500 MPa. Most often, the ratio of elastic modulus above and below the transition temperature is 20 or more.

The melting point and glass transition temperature of the hard segment are generally at least 10 degrees C., and preferably 20 degrees C., higher than the transition temperature of the soft segment. The transition temperature of the hard segment is preferably between -60 and 270 degrees C., and more often between 30 and 150 degrees C. The ratio by weight of the hard segment to soft segments is between about 5:95 and 95:5, and most often between 20:80 and 80:20. The shape memory polymers contain at least one physical crosslink (physical interaction of the hard segment) or contain covalent crosslinks instead of a hard segment. The shape memory polymers can also be interpenetrating networks or semi-interpenetrating networks.

Rapidly erodible polymers such as poly(lactide-co-glycolide)s, polyanhydrides, and polyorthoesters, which have carboxylic groups exposed on the external surface as the smooth surface of the polymer erodes, also can be used. In addition, polymers containing labile bonds, such as polyanhydrides and polyesters, are well known for their hydrolytic reactivity. Their hydrolytic degradation rates can generally be altered by simple changes in the polymer backbone and their sequence structure.

Examples of suitable hydrophilic polymers include but are not limited to poly(ethylene oxide), polyvinyl pyrrolidone, polyvinyl alcohol, poly(ethylene glycol), polyacrylamide poly(hydroxy alkyl methacrylates), poly(hydroxy ethyl methacrylate), hydrophilic polyurethanes, HYPAN, oriented HYPAN, poly(hydroxy ethyl acrylate), hydroxy ethyl cellulose, hydroxy propyl cellulose, methoxylated pectin gels, agar, starches, modified starches, alginates, hydroxy ethyl carbohydrates and mixtures and copolymers thereof.

Hydrogels can be formed from polyethylene glycol, polyethylene oxide, polyvinyl alcohol, polyvinyl pyrrolidone, polyacrylates, poly (ethylene terephthalate), poly(vinyl acetate), and copolymers and blends thereof. Several polymeric segments, for example, acrylic acid, are elastomeric only when the polymer is hydrated and hydrogels are formed. Other polymeric segments, for example, methacrylic acid, are crystalline and capable of melting even when the polymers are not hydrated. Either type of polymeric block can be used, depending on the desired application and conditions of use.

Curable materials include any material capable of being able to transform from a fluent or soft material to a harder material, by cross-linking, polymerization, or other suitable process. Materials may be cured over time, thermally, chemically, or by exposure to radiation. For those materials that are cured by exposure to radiation, many types of radiation may be used, depending upon the material. Wavelengths in the spectral range of about 100-1340 nm may be used. The material should absorb light within a wavelength range that is not readily absorbed by tissue, blood elements, physiological fluids, or water. Ultraviolet radiation having a wavelength ranging from about 100-400 nm may be used, as well as visible, infrared and thermal radiation. The following materials are some examples of curable materials: urethanes, polyurethane oligomer mixtures, acrylate monomers, aliphatic urethane acrylate oligomers, acrylamides, UV curable epoxies, photopolymerizable polyanhydrides and other UV curable monomers. Alternatively, the curable material can be a material capable of being chemically cured, such as silicone based compounds which undergo room temperature vulcanization. Alternatively, photocurable polyanhydrides solids are desirable. When cured at approximately 365 nm, less than 1% free monomer remains.

Some embodiments according to the invention comprise materials that are cured in a desired pattern. Such materials may be cured by any of the foregoing means. Further, for those materials that are photocurable, such a pattern may be created by coating the material in a negative image of the desired pattern with a masking material using standard photoresist technology. Absorption of both direct and incident radiation is thereby prevented in the masked regions, curing the device in the desired pattern. A variety ofbiocompatibly eroding coating materials may be used, including but not limited to gold, magnesium, aluminum, silver, copper, platinum, inconel, chrome, titanium indium, indium tin oxide. Projection optical photolithography systems that utilize the vacuum ultraviolet wavelengths of light below 240 nm provide benefits in terms of achieving smaller feature dimensions. Such systems that utilize ultraviolet wavelengths in the 193 nm region or 157 nm wavelength region have the potential of improving precision masking devices having smaller feature sizes.

Photopolymerization of multifunctional monomers readily allows for the production of high density crosslinked polymer networks having increased thermal stabililty, mechanical strenth, and resistance to solvent absorption. Additionally, photopolymerization can be performed in a matter of between seconds and minutes, conferring great clinical advantages, and control over the extent of polymerization desired. Endoprostheses fabricated utilizing photopolymerization thereby can transition rapidly and to a desired extent from a nearly liquid, highly flexible (and therefore easily tracked) form to a semi-rigid, stable, device exhibiting the requisite radial strength. Additionally, regions of endoprostheses can be selectively photopolymerized to achieve desired physical characteristics that vary from the physical characteristics of other regions of the device. For example, middle portions of an endoprosthesis where a high degree of structural rigidity is desired may be selectively photopolymerized to achieve a higher cross-linking density than the outer end portions, which may be desired to be more highly compliant. As another example, structural support members may be selectively photopolymerized to achieve greater structural rigidity than, for example, longitudinal connecting members. Materials may be selected for the wavelength at which they polymerize, and an endoprosthesis fabricated from materials at varied wavelengths disposed in regions according to the desired physical properties of the region. Examples of multifunctional monomers include diethylene glycol dimethacrylate, methacrylated 1,6-bis(carboxyphenoxy)hexane, and methacrylated pyromellitylimidoalanine.

Though not limited thereto, some embodiments according to the invention have been surface treated to comprise one or more therapeutic substances that will elute from the structure or prosthesis independently or as the material comprising the stent erodes. The diffusion coefficient of various regions of an endoprosthesis, for example, a luminal surface, may be varied according to the desired diffusion coefficient of a particular surface. Alternatively, therapeutic substances may be incorporated into the materials that comprise the endoprosthesis. According to the invention, such surface treatment and/or incorporation of therapeutic substances may be performed utilizing one or more of numerous processes that utilize carbon dioxide fluid, e.g., carbon dioxide in a liquid or supercritical state.

A supercritical fluid is a substance above its critical temperature and critical pressure (or "critical point"). Compressing a gas normally causes a phase separation and the appearance of a separate liquid phase. However, all gases have a critical temperature above which the gas cannot be liquefied by increasing pressure, and a critical pressure or pressure which is necessary to liquefy the gas at the critical temperature. For example, carbon dioxide in its supercritical state exists as a form of matter in which its liquid and gaseous states are indistinguishable from one another. For carbon dioxide, the critical temperature is about 31 degrees C (88 degrees D) and the critical pressure is about 73 atmospheres or about 7.38 MPa (1070 psi).

The term "supercritical carbon dioxide" as used herein refers to carbon dioxide at a temperature greater than about 31 degrees C and a pressure greater than about 7.38 MPa (1070 psi). Liquid carbon dioxide may be obtained at temperatures of from about -15 degrees C to about -55 degrees C and pressures of from about 0.53 MPa (77 psi) to about 2.31 MPa (335 psi). One or more solvents and blends thereof may optionally be included in the carbon dioxide. Illustrative solvents include, but are not limited to, tetrafluoroisopropanol, chloroform, tetrahydrofuran, cyclohexane, and methylene chloride. Such solvents are typically included in an amount, by weight, of up to about 20%.

In general, carbon dioxide may be used to effectively lower the glass transition temperature of a polymeric material to facilitate the infusion of pharmacological agent(s) into the polymeric material. Such agents include but are not limited to hydrophobic agents, hydrophilic agents and agents in particulate form. For example, following fabrication, an endoprosthesis and a hydrophobic pharmacological agent may be immersed in supercritical carbon dioxide. The supercritical carbon dioxide "plasticizes" the polymeric material, that is, it allows the polymeric material to soften at a lower temperature, and facilitates the infusion of the pharmacological agent into the polymeric endoprosthesis or polymeric coating of a stent at a temperature that is less likely to alter and/or damage the pharmacological agent.

As an additional example, an endoprosthesis and a hydrophilic pharmacological agent can be immersed in water with an overlying carbon dioxide "blanket". The hydrophilic pharmacological agent enters solution in the water, and the carbon dioxide "plasticizes" the polymeric material, as described above, and thereby facilitates the infusion of the pharmacological agent into a polymeric endoprosthesis or a polymeric coating of an endoprosthesis.

As yet another example, carbon dioxide may be used to "tackify", or render more fluent and adherent a polymeric endoprosthesis or a polymeric coating on an endoprosthesis to facilitate the application of a pharmacological agent thereto in a dry, micronized form. A membrane-forming polymer, selected for its ability to allow the diffusion of the pharmacological agent therethrough, may then applied in a layer over the endoprosthesis. Following curing by suitable means, a membrane that permits diffusion of the pharmacological agent over a predetermined time period forms.

In alternative embodiments of the present invention, at least one monomer or comonomer can be solubilized in carbon dioxide and copolymerized with a fluoromonomer. Any suitable monomers or comonomers can be employed, including, but not limited to, acrylate, methacrylate, acrylamide, methacrylamide, styrenics, ethylene, and vinyl ether monomers. The copolymerizations of the present invention may be carried out under temperature and pressure conditions similar to those given above.

Objectives of therapeutics substances incorporated into materials forming or coating an endoprosthesis according to the invention include reducing the adhesion and aggregation of platelets at the site of arterial injury, block the expression of growth factors and their receptors; develop competitive antagonists of growth factors, interfere with the receptor signaling in the responsive cell, promote an inhibitor of smooth muscle proliferation. Anitplatelets, anticoagulants, antineoplastics, antifibrins, enzymes and enzyme inhibitors, antimitotics, antimetabolites, anti-inflammatories, antithrombins, antiproliferatives, antibiotics, and others may be suitable.

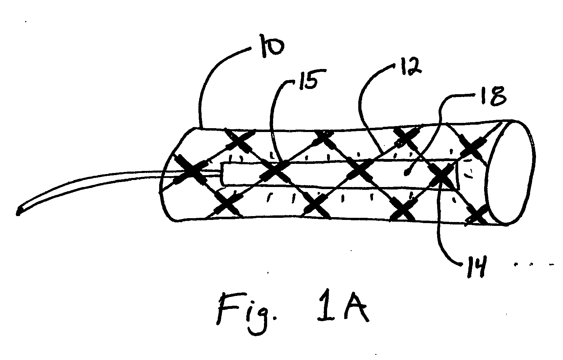

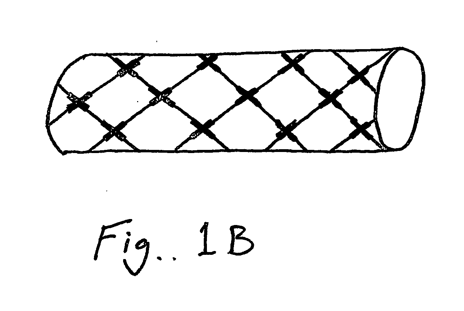

FIGS 1A-B represent plan views of another embodiment according to the invention. Endoprosthesis 10 of **FIGS 1A-B**, shown in its expanded configuration, comprises a generally tubular structure formed from one or more fibers 12. One or more fibers 12 comprise fiber points of intersection 14. Prior to deployment, fibers 12 comprise a photo-curable coating 15, at or near points of intersection 14, in semi-cured form. Following delivery and expansion of the endoprosthesis 10 by suitable means, the delivery catheter (not shown) is replaced by ultraviolet light delivery catheter 18. Ultraviolet radiation within the ranges discussed above is delivered via ultraviolet light delivery catheter 18, and photocurable coating 15 is cured. Ultraviolet light delivery catheter 18 is then removed from the vessel, and endoprosthesis 10 is left in place.

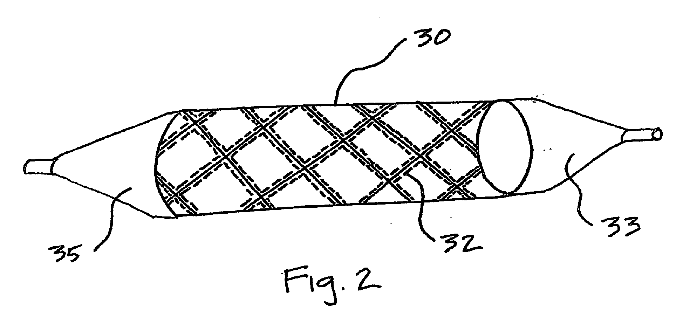

Alternatively, substantially the entire endoprosthesis may comprise a photocurable coating. As shown in **FIG. 2**, endoprosthesis 30 may be disposed on distal end 33 of expanded balloon 35, over which photolithographic masking material 32 has been applied in a pattern. Masking material 32 prevents the delivery of radiation, to leave desired portions, for example fiber points of intersection, exposed. Endoprosthesis 30 may then be exposed to ultraviolet or other suitable form of radiation, allowing the exposed portions of the coated device to cure. Following delivery of radiation, balloon 35 is removed. In time, the photolithographic masking material, and eventually endoprosthesis 30 may erode biocompatibly.

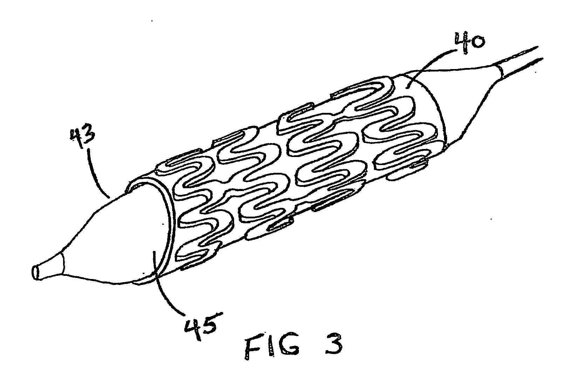

An alternative embodiment according to the invention is shown in **FIG. 3**. As discussed above with respect to FIG. 2, endoprosthesis 40 is mounted upon distal end 43 of balloon 45, which has been coated with photolithographic masking material 42 in a pattern such radiation is selectively delivered to endoprosthesis 40, allowing curing in selected regions of endoprosthesis 40.

In an alternative embodiment, an endoprosthesis can comprise multiple materials that are curable at different wavelengths, in order to confer varied physical properties on the prosthesis according to the desired properties of a particular region of the endoprosthesis. For example, the proximal and distal ends of a prosthesis can comprise one or more materials that are curable at a wavelength distinct from that at which the remainder of the prosthesis is curable, and can be selected for greater compliance. It has been shown clinically that restenosis occurs in response to vessel trauma at the proximal and distal ends of prostheses. By controlling the physical properties to enhance flexibility and to minimize compliance mismatch at the proximal and distal ends of the prosthesis, tremendous clinical benefit can be conferred upon the device. As a second example, the material selected to comprise the longitudinal connecting members can cure at a different wavelength than that at which the remainder of the prosthesis cures, to impart greater compliance and flexibility of longitudinal members while allowing the structural rigidity needed in support members.

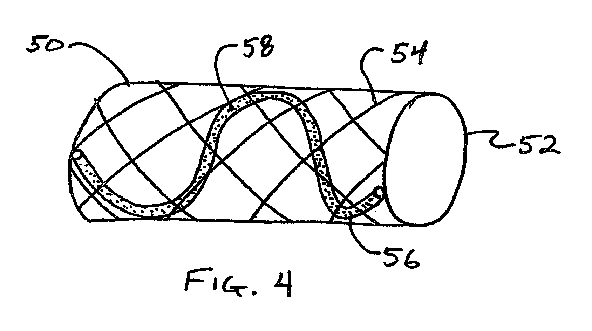

Turning now to **FIG 4**, a further embodiment according to the invention is provided. Endoprosthesis 50 comprises a generally tubular element 52. Although alternative configurations are possible, generally tubular element 52 is formed by weaving, as defined above, one or more fibers 54. Hollow element 56 is then woven or affixed to generally tubular element 52. Hollow element 56 comprises curable material 58 in its interior. Following expansion of endoprosthesis 50, curable material 58 is allowed to cure or, if it is photocurable, is exposed to radiation in order to initiate curing. Hollow element 56, following curing of curable material 58, confers structural support upon endoprosthesis 50.

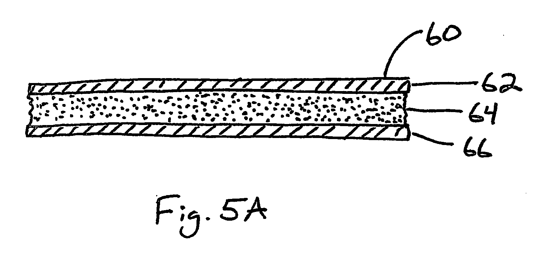

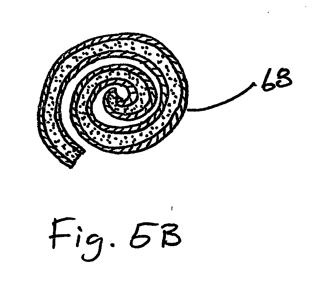

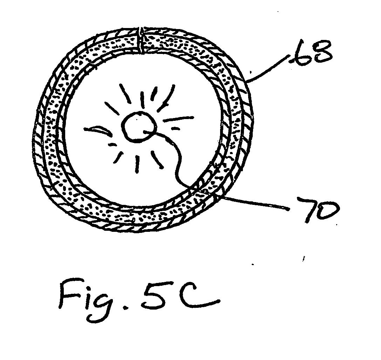

**FIGS. 5A-C** represent steps in the preparation of an alternative embodiment according to the invention. **FIG. 5A** is an enlarged end view of composite flat sheet 60. Composite flat sheet 60 comprises a first polymeric laminate layer 62, a photocurable and/or chemically reactive membrane 64, and second polymeric laminate layer 66. After formation of composite flat sheet 60, it is rolled to form unexpanded endoprosthesis 68, as shown in **FIG. 5C**. Upon expansion of endoprosthesis 68, light delivery source 70 is introduced within endoprosthesis 68, as seen in an end view in **FIG. 5C**. Photocurable and/or chemically reactive membrane 64 is thereby cured, conferring the requisite structural rigidity to endoprosthesis 68.

While particular forms of the invention have been illustrated and described above the foregoing descriptions are intended as examples, and to one skilled in the art it will be apparent that various modifications can be made without departing from the invention as defined by the claims.

## Claims

1. A system for treating a stenosis in a body lumen, said system comprising:
an endoprosthesis (10, 30, 40) comprising a first curable material (15);
means for selectively curing said first curable material,
wherein said first curable material is curable by exposure to a radiation source, said system further comprising a radiation source, wherein said radiation source comprises a radiation emitting catheter (18),
**characterised in that** said endoprosthesis further comprises a first region, said means for selectively curing said first curable material comprises a radiation emitting catheter comprising an inflatable balloon (35, 45), wherein said balloon comprises means to prevent the exposure of said first region of said endoprosthesis to radiation and wherein said means to prevent the exposure of said first region to radiation comprises a photolithographic coating (32).

2. The system of claim 1, wherein said photolithographic coating comprises a desired geometric pattern.

3. The system of claim 1 or 2 further comprising a second curable material that is curable by exposure to radiation, wherein said first curable material (15) cures at a first wavelength and said second curable material cures at a second wavelength.

## Patentansprüche

1. System zur Behandlung einer Stenose in einem Körperlumen, wobei das System umfasst:
eine Endoprothese (10, 30, 40), die ein erstes härtbares Material (15) umfasst,
Mittel zum selektiven Härten des ersten härtbaren Materials,
wobei das erste härtbare Material durch Einwirkung einer Strahlungsquelle härtbar ist, das System ferner eine Strahlungsquelle umfasst, wobei die Strahlungsquelle einen Strahlung emittierenden Katheter (18) umfasst,
**dadurch gekennzeichnet, dass** die Endoprothese ferner einen ersten Bereich umfasst, das Mittel zum selektiven Härten des ersten härtbaren Materials einen Strahlung emittierenden Katheter umfasst, der einen aufpumpbaren Ballon (35, 45) umfasst, wobei der Ballon Mittel umfasst, um die Einwirkung der Strahlung auf den ersten Bereich der Endoprothese zu verhindern, und wobei das Mittel, um die Einwirkung der Strahlung auf den ersten Bereich zu verhindern, eine fotolithografische Beschichtung (32) umfasst.

2. System nach Anspruch 1, wobei die fotolithografische Beschichtung ein gewünschtes geometrisches Muster umfasst.

3. System nach Anspruch 1 oder 2, das ferner ein zweites härtbares Material umfasst, das durch Einwirkung von Strahlung härtbar ist, wobei das erste härtbare Material (15) bei einer ersten Wellenlänge härtet und das zweite härtbare Material bei einer zweiten Wellenlänge härtet.

## Revendications

1. Système pour traiter une sténose dans une lumière corporelle, ledit système comprenant :
une endoprothèse (10, 30, 40) comprenant un premier matériau durcissable (15);
un moyen de durcir sélectivement ledit matériau durcissable,
dans lequel ledit matériau durcissable est durcissable par exposition à une source de rayonnement, ledit système comprenant en outre une source de rayonnement, dans lequel ladite source de rayonnement comprend un cathéter émetteur d'un rayonnement (18),
**caractérisé en ce que** ladite endoprothèse comprend en outre une première région, ledit moyen pour durcir sélectivement ledit matériau durcissable comprend un cathéter émetteur d'un rayonnement qui comprend un ballonnet gonflable (35, 45), dans lequel ledit ballonnet comprend un moyen d'empêcher l'exposition de ladite première région de ladite endoprothèse à un rayonnement, et dans lequel ledit moyen d'empêcher l'exposition de ladite première région au rayonnement comprend un revêtement photolithographique (32).

2. Système selon la revendication 1, dans lequel ledit revêtement photolithographique comprend un modèle géométrique souhaité.

3. Système selon la revendication 1 ou 2 comprenant en outre un second matériau durcissable qui est durcissable par exposition à un rayonnement, dans lequel ledit premier matériau durcissable (15) durcit à une première longueur d'onde et ledit second matériau durcit à une seconde longueur d'onde.
